# EUROPEAN PATENT SPECIFICATION

(11) **EP 1 613 327 B1**
(45) Date of publication and mention of the grant of the patent: **15.08.2007**
(21) Application number: 04725340.6
(22) Date of filing: 02.04.2004
(51) Int. Cl.: A61K 31/5415, A61K 45/06, A61P 13/08, A61K 31/18

(54) **PHARMACEUTICAL COMBINATION CONTAINING TAMSULOSIN AND A NON-STEROIDAL ANTI-INFLAMMATORY DRUG**
ZUSAMMENSETZUNG DIE TAMSULOSIN UND EIN NICHTSTEROIDES ENTZÜNDUNGSHEMMENDES MITTEL ENTHÄLT
COMBINAISON PHARMACEUTIQUE COMPRENANT DE LA TAMSULOSIN ET UN ANTI-INFLAMMATOIRE NON STÉROIDIEN

(30) Priority: 07.04.2003 DE 10315702
(43) Date of publication of application: 11.01.2006
(73) Proprietor: Boehringer Ingelheim International GmbH, 55216 Ingelheim am Rhein (DE); Boehringer Ingelheim Pharma GmbH & Co.KG, 55216 Ingelheim am Rhein (DE)
(72) Inventor: BAIKER, Wolfgang, 55546 Volxheim (DE); MEHLBURGER, Ludwig, 55411 BINGEN (DE)
(86) International application number: PCT/EP2004/003533
(87) International publication number: WO 2004/089379

(56) References cited:
- EP-A- 0 034 432
- WO-A-20/04026116
- DE-A- 10 024 752
- FR-A- 2 437 838
- GURUNADHA RAO TUNUGUNTLA H S ET AL: "Management of prostatitis" PROSTATE CANCER AND PROSTATIC DISEASES 2002 UNITED KINGDOM, vol. 5, no. 3, 2002, pages 172-179, XP008036511 ISSN: 1365-7852
- WAGENLEHNER F M E ET AL: "Therapy of prostatitis syndrome" UROLOGE - AUSGABE A 2001 GERMANY, vol. 40, no. 1, 2001, pages 24-28, XP008036514 ISSN: 0340-2592
- DATABASE BIOSIS [Online] BIOSCIENCES INFORMATION SERVICE, PHILADELPHIA, PA, US; 2003, DYAKOV V V ET AL: "Long-term treatment of chronic prostatitis with tamsulosin (omnik(R))." XP002300331 Database accession no. PREV200300132038 & UROLOGIYA (MOSCOW), no. 5, October 2002 (2002-10), pages 10-12,
- DUNN C J ET AL: "Tamsulosin: A review of its pharmacology and therapeutic efficacy in the management of lower urinary tract symptoms" DRUGS AND AGING 2002 NEW ZEALAND, vol. 19, no. 2, 2002, pages 135-161, XP008036515 ISSN: 1170-229X
- DELLABELLA M D ET AL: "Conservative managment of juxtavesical calculi with Tamsulosin." EUROPEAN UROLOGY SUPPLEMENTS, vol. 2, no. 1, February 2003 (2003-02), page 81, XP008036479 & XVIIITH CONGRESS OF THE EUROPEAN ASSOCIATION OF UROLOGY; MADRID, SPAIN; MARCH 12-15, 2003 ISSN: 1569-9056

## Description

The present invention relates to a pharmaceutical combination suitable for the treatment of prostatic hyperplasia or for the treatment of abacterial prostatitis.

### Background to the invention

BPH, a benign, non-cancerous enlargement of the prostate, is a well-known complaint in men, which generally comes to medical attention from the age of 50 onwards. About 50% of all men aged over 50 and 95% of all men aged over 70 are affected by it.

BPH is a generally progressive condition which in serious cases may endanger kidney function and require surgical intervention. The number of untreated patients runs at over 37 million worldwide. The growth of the prostate compresses or lengthens the urethra, causing the symptoms of urethral blockage and possibly leading to urinary retention.

The prostate consists of epithelial glandular tubes embedded in fibromuscular stroma. The hyperplastic growth of the prostate begins at about the age of 30 in the periurethrally located parts of the gland, the so-called transition zone. Apart from the effects of ageing, androgenic hormones constitute a crucial stimulus to growth in the post-pubertal regulation of the volume of the gland. In the normal prostate, the enzyme 5α-reductase in the epithelial cells converts the androgenic hormone testosterone (T) into dihydrotestosterone (DHT). DHT, an active androgenic prostate metabolite, binds to cytoplasmic receptors and is transported into the cell nucleus where it initiates RNA and protein synthesis and cell replication. It is assumed that BPH occurs in response to the effects of DHT on the ageing prostate and to changes in the stroma and epithelial cells (Steers, Zorn, Dis. Mon., 41 (7):437-497 (1995)).

Age-dependent changes in the serum concentrations of the hormonal regulatory circuit as a whole (LH, FSH, SHGB, T and DHT) and of other hormones which may affect this regulatory circuit (oestrogens, prolactin, testosterone derivatives), have been investigated as possible causes. However, there is no correlation between these age-dependent hormonal changes in the serum and the intraprostatic hormone concentrations. Thus, it is clear that the prostate itself is responsible for regulating the hormonal, milieu.

Possible points of attack for controlling the intraprostatic hormonal milieu are the 5α-reductase (i.e. the androgen metabolism), the hormone receptor expression in the epithelium and stroma, oestrogens and other hormones. In addition, numerous peptidal growth factors have a paracrine or autocrine effect on the local metabolism in the various compartments of the gland, by means of which the equilibrium of the cell kinetics can be shifted between proliferation and programmed cell death.

The clinical symptoms of BPH include both blocking symptoms (e.g. stoppage of the stream of urine, a weak or interrupted stream, urinary retention) which result directly from the constriction of the neck of the bladder and the prostatic urethra by the hyperplastic prostate, and also symptoms of an irritated lower urinary tract (e.g. urinary frequency, nycturia, dysuria, uresiesthesis, urinary incontinence). Untreated, BPH may lead to serious complications of the urinary tract and kidneys such as e.g. acute urinary retention and hydronephrosis (uronephrosis).

Tamsulosin hydrochloride is an α-receptor blocker and is used as a monosubstance for treating functional symptoms of prostatic hyperplasia (BPH). In addition, tamsulosin hydrochloride is also used as a monosubstance for treating abacterial prostatitis, as has been demonstrated by clinical trials, at least in sub-populations.

The synthesis of tamsulosin and the acid addition salts thereof was first described in European Patent No. EP 34432

A suitable sustained-release formulation is disclosed for example in US Patent No. 4,772,475.

Meloxicam or (4-hydroxy-2-methyl-N-(5-methyl-2-thiazolyl)-2H-1,2-benzothiazine-3-carboxamide-1,1-dioxide) belongs to the NSAIDs (non-steroidal anti-inflammatory drugs). It is known for its antirheumatic properties and is well tolerated by the stomach in the doses required for therapy. The active substance and its sodium salt as well as the N-methyl-D-glucamine salt (meglumine salt) thereof are described in EP 0 002 482 and its counterpart US Patent No. 4,233,299. The anti-inflammatory and pain-relieving properties of meloxicam also make this active substance very valuable for use in the treatment of pain.

Orally administered solid pharmaceutical preparations containing meloxicam, from which the active substance is rapidly released and reabsorbed, have been described previously (WO 99/49867), as well as orally administered syrup formulations (WO 99/49845) and highly concentrated stable solutions (WO 01/97813).

As for the mechanism of activity, it has been established that meloxicam is a cyclooxygenase-2 (COX-2) and oxidoreductase inhibitor.

Meloxicam is also known from US Patent No. 6,440,963 for the treatment of neuromuscular functional disorders of the lower urinary tract.

The combination of alpha blockers such as tamsulosin and an nonsteroidal anti inflammatory agent (ibuprofen or acetaminophen) for the treatment of prostatitis is disclosed in "Prostate Cancer and Prostatic Diseases, VoI.5(3), 2002, pages 172-179".
"Urologue, Vol.40(1), 2001, pages 24-28" discloses in context with the treatment of prostatitis a basic treatment with drugs such as alpha blockers (e.g. tamsulosin). It is mentioned that an nonsteroidal anti-inflammatory drug may be used in addition.
The abstract of BIOSIS (accession number no. PREV200300132038 (&Urologoya, 2002, pages 10-12) discloses the combination of tamsulosin and an antiinflammatory **therapy** for treating chronic prostatitis.

Surprisingly, the combination according to the invention of tamsulosin, an acid addition salt thereof, particularly tamsulosin hydrochloride, and meloxicam, the sodium or meglumine salt thereof, is particularly suitable for the treatment of benign prostatic hyperplasia or for the treatment of abacterial prostatitis.

The combination according to the invention contains both active substances in a formulation which contains between 0.1 and 1.5 mg of tamsulosin hydrochloride and between 0.1 and 20 mg of the non-steroidal inflammatory drug meloxicam or the sodium or meglumine salt thereof. A solid pharmaceutical combination containing 0.2 to 0.8 mg of tamsulosin hydrochloride and 1 to 7.5 mg of meloxicam or the sodium or meglumine salt thereof is preferred. The active substances may be administered orally.

Suitable conventional preparations include, for example, inert conventional carriers and/or diluents, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethyleneglycol, propyleneglycol, cetylstearylalcohol, carboxymethylcellulose or fatty substances such as hard fat or suitable mixtures thereof, incorporated in conventional galenic preparations such as plain or coated tablets, capsules, powders; suspensions or suppositories.

The active substances may be given orally in a wide variety of different dosage forms, for example, they may be formulated together with various pharmaceutically acceptable inert carriers in the form of tablets, capsules, pastilles, lozenges, hard sweets, powders, aqueous suspensions, elixirs, syrups and the like. Carriers of this kind comprise, for example, solid diluents or fillers, sterile aqueous media and various non-toxic organic solvents. In addition, oral formulations of this kind may be suitably sweetened and/or flavoured with various agents normally used for this purpose. Generally, the active substances are present in oral dosage forms of this kind in concentrations ranging from 0.5 % by weight to 90 % by weight; based on the total composition, in amounts sufficient to produce the desired dosage units. Other suitable dosage forms for the active substances include formulations for controlled release and devices which are well known to the specialists in the field.

Preferably, the two active substances are presented in a delayed-release formulation.

The invention is also suitable for the treatment of so-called "chronic abacterial benign prostatic hyperplasia".

According to the invention, the combination of the two active substances is suitable for both short-term and long-term therapy of benign prostatic hyperplasia.

According to the invention, the expression long-term therapy denotes a medical application of the combination of the two active substances in one formulation for at least 3 months or more.

The combination according to the invention appears to be especially suitable for treating a particularly enlarged prostate.

## Claims

1. Pharmaceutical combination containing a combination of the active substances tamsulosin or an acid addition salt thereof and meloxicam or the sodium or meglumine salt thereof.

2. Pharmaceutical combination according to one of claim 1, **characterised in that** it contains the meglumine salt of meloxicam.

3. Pharmaceutical combination according to one of claims 1 or 2, **characterised in that** it contains tamsulosin hydrochloride.

4. Pharmaceutical combination according to one of claims 1 to 3 for treating benign prostatic hyperplasia.

5. Pharmaceutical combination according to one of claims 1 to 3 for treating abacterial prostatitis.

## Patentansprüche

1. Pharmazeutische Kombination, enthaltend eine Kombination der Wirkstoffe Tamsulosin oder eines Säureadditionssalzes hiervon und Meloxicam oder das Natrium- oder Megluminsalz hiervon.

2. Pharmazeutische Kombination nach Anspruch 1, **dadurch gekennzeichnet, dass** sie das Megluminsalz von Meloxicam enthält.

3. Pharmazeutische Kombination nach einem der Ansprüche 1 oder 2, **dadurch gekennzeichnet, dass** es Tamsulosinhydrochlorid enthält.

4. Pharmazeutische Kombination nach einem der Ansprüche 1 bis 3 zur Behandlung von benigner Prostatahyperplasie.

5. Pharmazeutische Kombination nach einem der Ansprüche 1 bis 3 zur Behandlung abakterieller Prostatitis.

## Revendications

1. Combinaison pharmaceutique contenant une combinaison des substances actives tamsulosine ou un sel d'addition acide de celui-ci et le melixocam ou son sel de sodium ou de méglumine.

2. Combinaison pharmaceutique selon la revendication 1, **caractérisée en ce qu'**elle contient le sel de méglumine de meloxicam.

3. Combinaison pharmaceutique selon l'une des revendications 1 ou 2, **caractérisée en ce qu'**elle contient le chlorhydrate de tamsulosin.

4. Combinaison pharmaceutique selon l'une des revendications 1 à 3 pour le traitement de l'hyperplasie prostatique bénigne.

5. Combinaison pharmaceutique selon l'une des revendications 1 à 3 pour le traitement de la prostatite abactérienne.
